(19) Europäisches Patentamt
European Patent Office
Office européen des brevets

(11) Veröffentlichungsnummer: **0 075 172**
**B1**

(12) # EUROPÄISCHE PATENTSCHRIFT

(45) Veröffentlichungstag der Patentschrift: **29.05.85**

(51) Int. Cl.⁴: **C 07 C 145/02**, A 01 N 41/06

(21) Anmeldenummer: **82108189.0**

(22) Anmeldetag: **06.09.82**

(54) **N-Sulfenylierte Benzylsulfonamide, ein Verfahren und ihre Verwendung als Mikrobizide.**

(30) Priorität: **17.09.81 DE 3137061**

(43) Veröffentlichungstag der Anmeldung: **30.03.83 Patentblatt 83/13**

(45) Bekanntmachung des Hinweises auf die Patenterteilung: **29.05.85 Patentblatt 85/22**

(84) Benannte Vertragsstaaten: **AT BE CH DE FR GB IT LI NL SE**

(56) Entgegenhaltungen: **US - A - 4 208 348**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Kühle, Engelbert, Dr., Bodelschwinghstrasse 42, D-5060 Bergisch-Gladbach (DE)**
Erfinder: **Paulus, Wilfried, Dr., Deswatinesstrasse 90, D-4150 Krefeld (DE)**
Erfinder: **Brandes, Wilhelm, Dr., Eichendorffstrasse 3, D-5623 Leichlingen (DE)**
Erfinder: **Genth, Hermann, Dr., Am Heckerhof 60, D-4150 Krefeld (DE)**
Erfinder: **Paul, Volker, Dr., Ahornstrasse 5, D-5650 Solingen (DE)**
Erfinder: **Reinecke, Paul, Dr., Lessingstrasse 11, D-5090 Leverkusen (DE)**

**EP 0 075 172 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft neue N-sulfenylierte Benzylsulfonamide, ein Verfahren zu ihrer Herstellung und ihre Verwendung als Fungizide und Mikrobizide.

Seit langem sind Schwermetallsalze der Ethylen-1,2-bisdithiocarbaminsäure in Landwirtschaft und Gartenbau zur Bekämpfung von pflanzenpathogenen Pilzen in Gebrauch (R. Wegler, Chemie der Pflanzenschutz- und Schädlingsbekämpfungsmittel, Band 2, S. 65, Springer Verlag Berlin, Heidelberg, New York 1970).

Weiterhin ist seit langem bekannt, daß N-Trihalogenmethylthio-Verbindungen als Fungizide in Landwirtschaft und Gartenbau verwendet werden können. So werden z. B. N-(Trichlormethylthio)-tetrahydrophthalimid (DE-PS 887 506) und N,N-Dimethyl-N'-phenyl-N'-(fluordichlormethylthio)-sulfamid (Angew. Chem. 76, 807 (1964)) im Obst- und Weinbau zur Bekämpfung von Pilzkrankheiten praktisch eingesetzt. Letztere Verbindung zeigt darüber hinaus eine Wirkung im Holzschutz zur Bekämpfung holzverfärbender Pilze (R. Wengler, Chemie der Pflanzenschutz- und Schädlingsbekämpfungsmittel, Bd. 4, S. 269 (1977), Springer-Verlag Berlin).

Die Wirkung dieser Verbindungen ist jedoch, insbesondere bei niedrigen Aufwandmengen, nicht immer ganz befriedigend.

Es wurden neue N-sulfenylierte Benzylsulfonamide der allgemeinen Formel I

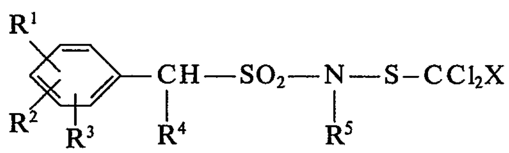

(I)

gefunden, in welcher

$R^1$, $R^2$ und $R^3$ unabhängig voneinander für Wasserstoff, Halogen, Nitro, Alkyl und Halogenalkyl stehen und

$R^4$ Wasserstoff oder Alkyl bedeuten,

$R^5$ für Alkyl mit 1 bis 6 C-Atomen, Alkenyl mit 2 bis 6 C-Atomen steht, wobei diese Reste gegebenenfalls durch Heteroatome wie Sauerstoff oder Schwefel ein oder mehrmals unterbrochen sein können, Cycloalkyl mit 3 bis 7 C-Atomen, Aralkyl mit 1 bis 4 C-Atomen im Alkylteil und 6 bis 10 C-Atomen im Arylteil, Phenyl und Naphthyl steht, wobei Aralkyl, Phenyl und Naphthyl gegebenenfalls mit Halogen, Nitro, Cyano, Methyl, Trifluormethyl, Alkoxy und Alkylthio mit 1 bis 4 C-Atomen substituiert sein können und

X Halogen bedeutet.

Man erhält die N-sulfenylierten Benzylsulfonamide der allgemeinen Formel I

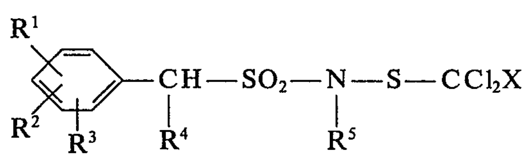

(I)

in welcher

$R^1$, $R^2$ und $R^3$ unabhängig voneinander für Wasserstoff, Halogen, Nitro, Alkyl und Halogenalkyl stehen und

$R^4$ Wasserstoff oder Alkyl bedeutet,

$R^5$ für Alkyl mit 1 bis 6 C-Atomen, Alkenyl mit 2 bis 6 C-Atomen steht, wobei diese Reste gegebenenfalls durch Heteroatome wie Sauerstoff oder Schwefel ein oder mehrmals unterbrochen sein können, Cycloalkyl mit 3 bis 7 C-Atomen, Aralkyl mit 1 bis 4 C-Atomen im Alkylteil und 6 bis 10 C-Atomen im Arylteil, Phenyl und Naphthyl steht, wobei Aralkyl, Phenyl und Naphthyl gegebenenfalls mit Halogen, Nitro, Cyano, Methyl, Trifluormethyl, Alkoxy und Alkylthio mit 1 bis 4 C-Atomen substituiert sein können und

X Halogen bedeutet,

wenn man Benzylsulfonamide der allgemeinen Formel II

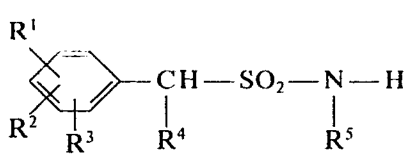

(II)

in welcher

$R^1$ bis $R^5$ die oben angegebene Bedeutung haben,

mit einem Sulfenylchlorid der Formel III

$$Cl{-}S{-}CCl_2X \quad (III)$$

in welcher

X die oben angegebene Bedeutung hat,

in Gegenwart eines säurebindenden Mittels und gegebenenfalls in Gegenwart eines Verdünnungsmittels, umsetzt.

Die neuen N-sulfenylierten Benzylsulfonamide weisen starke fungizide und mikrobizide Eigenschaften auf.

Überraschenderweise besitzen die erfindungsgemäßen N-sulfenylierten Benzylsulfonamide in verschiedenen Kulturen, z. B. in Reis, eine höhere fungizide Wirksamkeit als die bekannten N-Trihalogenmethylthio-Verbindungen. Außerdem zeigen sie eine höhere Aktivität bei holzzerstörenden Pilzen als die bekannten Verbindungen.

Von den erfindungsgemäßen N-sulfenylierten Benzylsulfonamiden der Formel I sind bevorzugt diejenigen, bei denen

$R^1$, $R^2$ und $R^3$ unabhängig voneinander für Wasserstoff, Chlor, Brom, Nitro, Alkyl mit 1 bis 4 C-Atomen, Halogenalkyl mit 1 bis 4 C-Atomen und 1 bis 5 Halogenatomen stehen,
$R^4$ für Wasserstoff oder Alkyl mit 1 bis 4 C-Atomen steht,
$R^5$ für Alkyl mit 1 bis 6 C-Atomen, Alkenyl mit 2 bis 6 C-Atomen steht, wobei diese Reste gegebenenfalls durch Heteroatome wie Sauerstoff oder Schwefel ein oder mehrmals unterbrochen sein können, Cycloalkyl mit 3 bis 7 C-Atomen, Aralkyl mit 1 bis 4 C-Atomen im Alkylteil und 6 bis 10 C-Atomen im Arylteil, Phenyl und Naphthyl steht, wobei Aralkyl, Phenyl und Naphthyl gegebenenfalls mit Halogen, Nitro, Cyano, Methyl, Trifluormethyl, Alkoxy und Alkylthio mit 1 bis 4 C-Atomen substituiert sein können und
X für Fluor oder Chlor steht.

Besonders bevorzugt sind die N-sulfenylierten Benzylsulfonamide der Formel I, bei denen

$R^1$ und $R^2$ unabhängig voneinander für Wasserstoff, Chlor, Nitro, Methyl und Trifluormethyl stehen,
$R^3$ für Wasserstoff steht,
$R^4$ für Wasserstoff steht und
$R^5$ für Alkyl mit 1 bis 6 C-Atomen, Alkenyl mit 2 bis 6 C-Atomen steht, die gegebenenfalls durch Heteroatome wie Sauerstoff oder Schwefel ab dem zweiten C-Atom ein- oder mehrmals unterbrochen sein können, für Cycloalkyl mit 3 bis 7 C-Atomen steht, das gegebenenfalls mit Alkyl mit 1 bis 4 C-Atomen substituiert sein kann, für Benzyl und für Phenyl steht, die beide gegebenenfalls mit Fluor, Chlor, Brom, Nitro, Cyano, Methyl, Trifluormethyl, Alkoxy und Alkylthio mit 1 bis 4 C-Atomen substituiert sein können und
X für Fluor steht.

Im einzelnen seien neben den Herstellungsbeispielen folgende N-sulfenylierte Benzylsulfonamide der Formel I genannt:

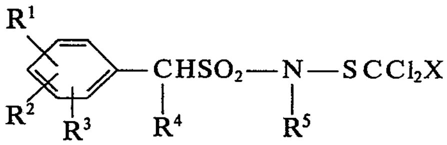

(I)

| $R^1$ | $R^2$ | $R^3$ | $R^4$ | $R^5$ | X |
|---|---|---|---|---|---|
| H | H | H | H | $-CH_2-CH=CH_2$ | F |
| H | H | H | H | $-CH_2CH_2OCH_3$ | F |
| H | H | H | H | $-CH_2CH_2SC_2H_5$ | F |
| H | H | H | H | 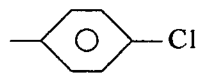 | F |
| H | H | H | H | 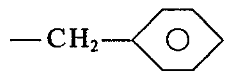 | F |
| 4-Cl | H | H | H | 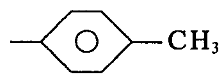 | F |
| 4-Cl | H | H | H | $-CH_3$ | Cl |
| $4-CF_3$ | H | H | H | $-C_4H_9$-tert. | F |
| $4-CF_3$ | 2-Cl | H | H | $-CH_3$ | F |
| $4-CF_3$ | H | H | H | $-CH_2-CH=CH_2$ | F |
| 4-Cl | 2-Cl | 5-Cl | H | $-CH_3$ | F |
| $3-NO_2$ | H | H | H | $-C_2H_5$ | F |
| 2-Cl | 6-Cl | H | H | $-C_3H_7i$ | F |
| H | H | H | $CH_3$ | $-CH_3$ | F |
| H | H | H | $CH_3$ | 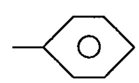 | F |

Verwendet man beispielsweise 4-Chlorbenzylsulfo-N-methylamid und Fluordichlormethansulfenylchlorid als Ausgangskomponenten, so kann der Reaktionsverlauf durch das nachfolgende Formelschema wiedergegeben werden:

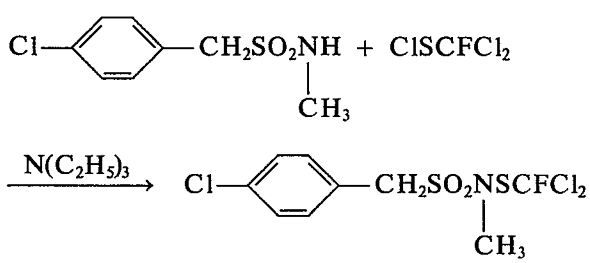


Die als Ausgangsstoffe benötigten Benzylsulfonylsäureamide sind durch die Formel II allgemein definiert. In dieser Formel haben $R^1$ bis $R^5$ vorzugsweise und besonders bevorzugt die Bedeutungen, die bei der Beschreibung der erfindungsgemäßen Verbindungen der Formel I für diese Substituenten bereits vorzugsweise und besonders bevorzugt genannt worden sind.

Die Benzolsulfonamide sind bekannt oder in an sich bekannter Weise erhältlich, wenn man Benzylsulfochloride der Formel IV

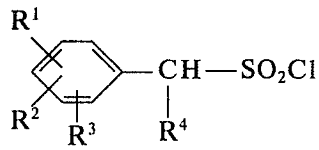

(IV)

in welcher

$R^1$ bis $R^4$ die bei Formel I angegebene Bedeutung haben,

mit einem primären Amin der Formel V

$$R^5{-}NH_2 \qquad (V)$$

in welcher

$R^5$ die oben angegebene Bedeutung hat,

gegebenenfalls in Gegenwart einer Base und eines Verdünnungsmittels umsetzt.

(Vergleiche Houben-Weyl, Methoden der organischen Chemie, 4. Auflage, Band 9, Seite 395 (1955).)

Die als Ausgangsstoffe benötigten Benzylsulfonylchloride sind durch Formel IV allgemein definiert. In dieser Formel stehen $R^1$ bis $R^4$ vorzugsweise und besonders bevorzugt für die Bedeutungen, die bereits im Zusammenhang mit Formel I vorzugsweise und besonders bevorzugt genannt worden sind.

Geeignet Benzylsulfochloride der Formel IV sind beispielsweise Benzyl-, 4-Methyl-, 4-Chlor-, 2,4-Dichlor-, 4-Nitro-, 4-Fluor-, 3-Trifluormethylbenzylsulfochlorid. Die Verbindungen sind bekannt oder in an sich bekannter Weise aus entsprechenden Benzylchloriden zugänglich (vgl. Houben-Weyl, Methoden der organischen Chemie, 4. Auflage, Band 9, Seite 395 (1955)).

Die als Ausgangsstoffe benötigten Amine sind durch Formel V allgemein definiert. In dieser Formel steht $R^5$ vorzugsweise und besonders bevorzugt für die Bedeutungen, die bereits in Zusammenhang mit Formel I vorzugsweise und besonders bevorzugt genannt worden sind.

Geeignete Amine der Formel V sind beispielsweise Methyl-, Ethyl-, Isopropyl-, Allyl-, tert.-Butyl-, Methoxyethyl-, Methylmercaptoethyl-, Cyclopentyl-, Cyclohexyl-, Benzyl-, 4-Nitrobenzylamin, Anilin, 4-Chloranilin, 3-Fluoranilin, 2-Toluidin, 3-Chlor-4-trifluormethylanilin und 1-Naphthylamin. Sie sind bekannte Verbindungen.

Verwendet man beispielsweise 4-Chlor-benzylsulfochlorid und Methylamin als Ausgangsstoffe, so kann der Reaktionsverlauf durch das folgende Formelschema wiedergegeben werden:

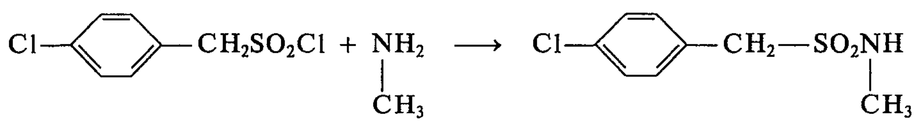

Bei der Durchführung des Verfahrens kommen als Verdünnungsmittel alle inerten organischen Lösungsmittel in Frage. Hierzu gehören vorzugsweise Kohlenwasserstoffe wie Toluol, Chlorkohlenwasserstoffe wie Methylenchlorid und Chlorbenzol, oder Ether wie Dioxan.

Als säurebindende Mittel können anorganische Basen wie Natriumhydroxid und Natriumcarbonat oder tert.-Amine wie Pyridin und Triethylamin verwendet werden.

Pro Mol Benzolsulfochlorid der Formel IV wird etwa 1 Mol primäres Amin der Formel V eingesetzt.

Die Reaktionstemperaturen können in einem größeren Bereich variiert werden. Im allgemeinen arbeitet man von 0 bis 100°C, vorzugsweise von 20 bis 50°C.

Das Verfahren kann wie folgt durchgeführt werden: Das Benzylsulfochlorid der Formel IV wird in einem Verdünnungsmittel vorgelegt und auf 40°C erhitzt. Dazu wird das primäre Amin der Formel V portionsweise gegeben. Isolierung und Reinigung des Benzylsulfonamids der Formel III erfolgt auf übliche Art und Weise.

Als Sulfenylchloride der Formel III können Fluordichlormethan-sulfenylchlorid und Trichlormethansulfenylchlorid eingesetzt werden.

Bei der Durchführung des erfindungsgemäßen Verfahrens kommen als Verdünnungsmittel alle inerten organischen Lösungsmittel in Frage. Hierzu gehören vorzugsweise Kohlenwasserstoffe wie Toluol, Chlorkohlenwasserstoffe wie Methylenchlorid und Chlorbenzol, oder Ether wie Dioxan und Wasser.

Als säurebindende Mittel können anorganische Basen wie Natriumhydroxid und Natriumcarbonat oder tert.-Amine wie Pyridin und Triethylamin verwendet werden.

Die Reaktionstemperaturen können in einem größeren Bereich variiert werden. Im allgemeinen arbeitet man von 0 bis 100°C, vorzugsweise von 20 bis 50°C.

Das erfindungsgemäße Verfahren wird wie folgt ausgeführt: Ein Benzolsulfonsäureamid der Formel III und ein Sulfenylchlorid der Formel III wird in einem Verdünnungsmittel vorgelegt. Dazu wird bei Raumtemperatur der Säurebinder portionsweise gegeben, so daß die Reaktionstemperatur auf etwa 40°C ansteigt. Nach Beendigung der Umsetzung wird das N-sulfenylierte Benzylsulfonamid der Formel (I) mit Wasser ausgefällt und auf übliche Art isoliert und gereinigt.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Darüber hinaus zeigen die erfindungsgemäßen Wirkstoffe bakterizide und akarizide Eigenschaften.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Weiterhin können die erfindungsgemäßen Wirkstoffe zur Bekämpfung von Mikroorganismen in technischen Materialien verwendet werden.

Technische Materialien, die durch die erfindungsgemäßen Substanzen vor einer mikrobiellen Veränderung und Zerstörung geschützt werden sollen, sind beispielsweise Klebstoffe, Leime, Papiere und Kartone, Textilien, Leder, Holz, Anstrichmittel und Kunststoffartikel, die von Mikroorganismen befallen und zersetzt werden können.

Mikroorganismen, die einen Abbau oder eine Veränderung der technischen Materialien bewirken können, sind beispielsweise Bakterien, Pilze, Hefen, Algen, Schleime und Viren. Vorzugsweise wirken die erfindungsgemäßen Substanzen gegen Schimmelpilze, holzverfärbende Pilze und holzzerstörende Pilze (Basidiomyceten) sowie gegen Schleimorganismen.

Es seien beispielsweise Mikroorganismen der folgenden Gattungen genannt:

Alternaria, wie Alternaria tenuis,
Aspergillus, wie Aspergillus niger,
Chaetomium, wie Chaetomium globosum,
Coniophora, wie Coniophora cerebella,
Lentinus, wie Lentinus tigrinus,
Penicillium, wie Penicillium glaucum,
Polyporus, wie Polyporus versicolor,
Pullularia, wie Pullularia pullulans,
Sclerophoma, wie Sclerophoma pityophila,
Staphylococcus, wie Staphylococcus aureus.

Je nach ihrem Anwendungsgebiet können die erfindungsgemäßen Substanzen in die üblichen Formulierungen überführt werden, wie Lösungen, Emulsionen, Suspensionen, Pulver, Pasten und Granulate.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B.Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgiermittel und/oder schaumerzeugende Mittel kommen in Frage: z. B. nichtionogene und anionische Emulgatoren, wie Polyoxyethylen-Fettsäure-Ester, Polyoxyethylen-Fettalkohol-Ether, z. B. Alkylarylpolyglykol-ether, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln angewendet werden.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können wie Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

Die erfindungsgemäßen neuen Wirkstoffe können auch in Mischung mit anderen bekannten Wirkstoffen vorliegen. Beispielsweise seien die folgenden Wirkstoffe genannt: Benzimidazolyl-methylcarbamate, Tetramethyl-thiuramdisulfid, Zn-Salze von Dialkyldithiocarbamaten, 2,4,5,6-Tetrachlor-isophthalonitril, Thiazolylbenzimidazol, Mercaptobenzthiazol und Phenolderivate, wie 2-Phenylphenol, (2,2'-Dihydroxy-5,5'-dichlor)-diphenylmethan.

## Herstellungsbeispiele

1) 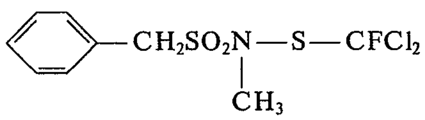

13 g (0,07 Mol) Benzylsulfomethylamid werden in 100 ml Dioxan unter Zusatz von 13 g (0,077 Mol) Fluordichlormethansulfenchlorid gelöst. In diese Lösung tropft man 7,8 g (0,077 Mol) Triethylamin und läßt die Temperatur bis etwa 40° ansteigen. Man rührt eine Zeitlang und fällt das Produkt mit Wasser aus. Ausbeute 16 g = 70% der Theorie. Aus Toluol-Petrolether Fp. 91—92°.

In analoger Weise erhält man folgende Verbindungen:

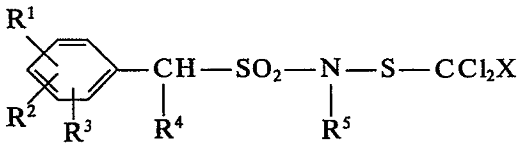

(I)

| Bsp. | $R^1$ | $R^2$ | $R^3$ | $R^4$ | $R^5$ | X | Phys. Konst. |
|---|---|---|---|---|---|---|---|
| 2 | H | H | H | H | $C_2H_5$ | F | 59–61°C |
| 3 | H | H | H | H | i-$C_3H_7$ | F | 80–81°C |
| 4 | H | H | H | H | n-$C_3H_7$ | F | $n_D^{20}$ 1,5434 |
| 5 | H | H | H | H | $C_3H_5$ | F | 74–76°C |
| 6 | H | H | H | H | n-$C_4H_9$ | F | $n_D^{20}$ 1,5381 |
| 7 | H | H | H | H | t-$C_4H_9$ | F | 65–68°C |
| 8 | 2-Cl | H | H | H | $C_6H_5$ | F | 117–120°C |
| 9 | 4-Cl | H | H | H | $CH_3$ | F | 98–100°C |
| 10 | 4-Cl | H | H | H | $C_2H_5$ | F | 57°C |
| 11 | 4-Cl | H | H | H | i-$C_3H_7$ | F | 115°C |
| 12 | 4-Cl | H | H | H | t-$C_4H_9$ | F | $n_D^{20}$ 1,5529 |
| 13 | 4-Cl | H | H | H | $C_6H_{11}$ | F | 130°C |
| 14 | 4-Cl | H | H | H | $C_6H_5$ | F | 118–122°C |
| 15 | 4-Cl | 3-Cl | H | H | $CH_3$ | F | 78–81°C |
| 16 | 4-Cl | 3-Cl | H | H | $C_6H_5$ | F | 116–119°C |
| 17 | 6-Cl | 2-Cl | H | H | $C_6H_5$ | F | 190–196°C |
| 18 | 2-$CF_3$ | H | H | H | $CH_3$ | F | $n_D^{20}$ 1,5178 |
| 19 | 2-$CF_3$ | H | H | H | $C_6H_5$ | F | 134–136°C |
| 20 | 4-$NO_4$ | H | H | H | $CH_3$ | F | 122°C |
| 21 | Cl | $NO_2$ | H | H | $C_6H_5$ | F | 99–102°C |

I) Herstellung der Vorprodukte

V 1 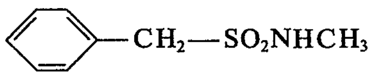

20 g Benzylsulfochlorid werden in 100 ml Toluol mit gasförmigem Methylamin 40° versetzt. Das zusammen mit dem Methylaminhydrochlorid anfallende Reaktionsprodukt wird in der Kälte abgesaugt und mit Wasser gewaschen. Ausbeute 13 g, Fp. 118–199°.

Analog werden folgende Sulfonamide hergestellt:

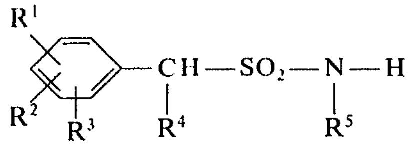

(II)

| Bsp. | $R^1$ | $R^2$ | $R^3$ | $R^4$ | $R^5$ | Schmp. °C |
|---|---|---|---|---|---|---|
| V 2 | H | H | H | H | $C_2H_5$ | ölig |
| V 3 | H | H | H | H | i-$C_3H_7$ | 98–100° |
| V 4 | H | H | H | H | n-$C_3H_7$ | 95–96° |
| V 5 | H | H | H | H | $C_3H_5$ | 74–75° |
| V 6 | H | H | H | H | n-$C_4H_9$ | 96–97° |
| V 7 | H | H | H | H | t-$C_4H_9$ | 102–103° |
| V 8 | 2-Cl | H | H | H | $C_6H_5$ | 98° |
| V 9 | 4-Cl | H | H | H | $CH_3$ | 108–110° |
| V 10 | 4-Cl | H | H | H | $C_2H_5$ | 96–97° |
| V 11 | 4-Cl | H | H | H | i-$C_3H_7$ | 138–140° |
| V 12 | 4-Cl | H | H | H | t-$C_4H_9$ | 141° |
| V 13 | 4-Cl | H | H | H | $C_6H_{11}$ | 168–169° |
| V 14 | 4-Cl | H | H | H | $C_6H_5$ | 89° |
| V 15 | 4-Cl | 3-Cl | H | H | $CH_3$ | 117° |
| V 16 | 4-Cl | 3-Cl | H | H | $C_6H_5$ | 129–132° |
| V 17 | 6-Cl | 2-Cl | H | H | $C_6H_5$ | 179° |
| V 18 | 2-$CF_3$ | H | H | H | $CH_3$ | 96–98° |
| V 19 | 2-$CF_3$ | H | H | H | $C_6H_5$ | 107–109° |
| V 20 | 4-$NO_2$ | H | H | H | $CH_3$ | 141–143° |
| V 21 | Cl | $NO_2$ | H | H | $C_6H_5$ | 115–119° |

## Anwendungsbeispiele

### Beispiel A

Leptosphaeria nodorum-Test (Weizen)/protektiv

Lösungsmittel: 100 Gew.-Teile Dimethylformamid
Emulgator: 0,25 Gew.-Teile Alkylarylpolyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100% relativer Luftfeuchtigkeit in einer Inkubationskabine.

Die Pflanzen werden in einem Gewächshaus bei einer Temperatur von ca. 15°C und einer relativen Luftfeuchtigkeit von ca. 80% aufgestellt.

10 Tage nach der Inokulation erfolgt die Auswertung.

Eine deutliche Überlegenheit in der Wirksamkeit gegenüber den Stand der Technik zeigen bei diesem Test z. B. die Verbindungen gemäß folgender Herstellungsbeispiele: 9, 15, 2, 3, 4, 5, 6.

## Beispiel B

### Botrytis-Test (Bohne)/protektiv

Lösungsmittel: 4,7 Gew.-Teile Aceton
Emulgator: 0,3 Gew.-Teile Alkylarylpolyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Eine deutliche Überlegenheit in der Wirksamkeit gegenüber dem Stand der Technik zeigen in diesem Test z. B. die Verbindungen gemäß folgender Herstellungsbeispiele: 20, 9.

## Beispiel C

### Phytophthora-Test (Tomate)/protektiv

Lösungsmittel: 4,7 Gew.-Teile Aceton
Emulgator: 0,3 Gew.-Teile Alkylarylpolyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100% relativer Luftfeuchtigkeit und ca. 20°C aufgestellt.

3 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele: 1, 9, 15, 2, 3, 4, 5, 6.

## Beispiel D

### Puccinia-Test (Weizen)/protektiv

Lösungsmittel: 100 Gew.-Teile Dimethylformamid
Emulgator: 0,25 Gew.-Teile Alkylarylpolyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1%igen wäßrigen Agarlösung inokuliert. Nach Antrocknen besprüht man die Pflanzen mit der Wirkstoffzubereitung taufeucht. Die Pflanzen verbleiben 24 Stunden bei 20°C und 100% rel. Luftfeuchtigkeit in einer Inkubationskabine.

Die Pflanzen werden in einem Gewächshaus bei einer Temperatur von ca. 20°C und einer relativen

Luftfeuchtigkeit von ca. 80% aufgestellt, um die Entwicklung von Rotpusteln zu begünstigen.

10 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele: 1.

Beispiel E

Tilletia caries-Test (Weizen)/Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das Saatgut, das vorher mit 5 g Chlamydosporen von Tilletia caries pro kg Saatgut kontaminiert worden ist, 3 Minuten lang mit dem Beizmittel in einer verschlossenen Glasflasche.

Das Saatgut setzt man auf feuchtem Lehm unter einer Deckschicht aus einer Lage Mull und 2 cm feuchtem Vermiculit im Kühlschrank 10 Tage bei 10°C optimalen Keimungsbedingungen für die Sporen aus.

10 Tage nach der Aussaat erfolgt die Auswertung auf Sporenkeimung auf den Weizenkörnern.

Eine deutliche Überlegenheit in der Wirksamkeit gegenüber dem Stand der Technik zeigen bei diesem Test z. B. die Verbindungen gemäß folgender Herstellungsbeispiele: 1.

Beispiel F

Pyricularia-Test (Reis)/protektiv

Lösungsmittel: 12,5 Gew.-Teile Aceton
Emulgator: 0,3 Gew.-Teile Alkylarylpolyglykolether

Zur Herstellung einer zweckmäßigen Wirkstoffzubereitung vermischt man 1 Gew.-Teil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100% rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen bei diesem Test z. B. die Verbindungen gemäß folgender Herstellungsbeispiele: 20, 9, 18, 5, 6.

Beispiel G

Zum Nachweis der Wirksamkeit gegen Pilze werden die minimalen Hemm-Konzentrationen (MHK) von erfindungsgemäßen Wirkstoffen bestimmt:

Ein Agar, der aus Bierwürze und Pepton hergestellt wird, wird mit erfindungsgemäßen Wirkstoffen in Konzentrationen von 0,1 mg/l bis 5000 mg/l versetzt. Nach Erstarren des Agars erfolgt Kontamination mit Reinkulturen der in der Tabelle aufgeführten Testorganismen. Nach 2wöchiger Lagerung bei 28°C und 60 bis 70% relativer Luftfeuchtigkeit wird die MHK bestimmt. MHK ist die niedrigste Konzentration an Wirkstoff, bei der keinerlei Bewuchs durch die verwendete Mikrobenart erfolgt, sie ist in der nachstehenden Tabelle angegeben.

Bei diesem Test werden Verbindungen gemäß folgender Herstellungsbeispiele verwendet: 9.

Beispiel H

Wirkung auf Schleimorganismen

Die Verbindung gemäß Herstellungsbeispiel 9 wird in Konzentrationen von jeweils 0,1 bis 100 mg/l in Allens Nährlösung (Arch. Mikrobiol. 17, 34 bis 53 (1952)), die in 4 l sterilem Wasser, 0,2 g Ammoniumchlorid, 4,0 g Natriumnitrat, 1,0 Dikaliumhydrogenphosphat, 0,2 g Calciumchlorid, 2,05 g Magnesiumsulfat, 0,02 g Eisenchlorid und 1% Caprolactam enthält, in wenig Aceton gelöst, zur Anwendung gebracht. Kurz vorher wird die Nährlösung mit Schleimorganismen (ca. $10^6$ Keime/ml), die

aus bei der Polyamid-Herstellung verwendeten Spinnwasser-Kreisläufen isoliert wurden, infiziert. Nährlösungen, die die minimale Hemmkonzentration (MHK) oder größere Wirkstoffkonzentrationen aufweisen, sind nach 3wöchiger Kultur bei Raumtemperatur noch völlig klar, d. h. die in wirkstofffreien Nährlösungen nach 3 bis 4 Tagen bemerkbare starke Vermehrung der Mikroben und Schleimbildung unterbleibt.

Für das Benzylsulfonamid gemäß Herstellungsbeispiel 9 wurde auf diese Weise die MHK ermittelt.

## Patentansprüche

1. N-sulfenylierte Benzylsulfonamide der allgemeinen Formel I

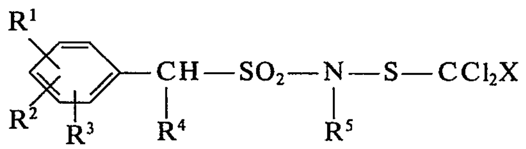

(I)

in welcher

$R^1$, $R^2$ und $R^3$ unabhängig voneinander für Wasserstoff, Halogen, Nitro, Alkyl und Halogenalkyl stehen und
$R^4$ Wasserstoff oder Alkyl bedeutet,
$R^5$ für Alkyl mit 1 bis 6 C-Atomen, Alkenyl mit 2 bis 6 C-Atomen steht, wobei diese Reste gegebenenfalls durch Heteroatome wie Sauerstoff oder Schwefel ein oder mehrmals unterbrochen sein können, Cycloalkyl mit 3 bis 7 C-Atomen, Aralkyl mit 1 bis 4 C-Atomen im Alkylteil und 6 bis 10 C-Atomen im Arylteil, Phenyl und Naphthyl steht, wobei Aralkyl, Phenyl und Naphthyl gegebenenfalls mit Halogen, Nitro, Cyano, Methyl, Trifluormethyl, Alkoxy und Alkylthio mit 1 bis 4 C-Atomen substituiert sein können und
X Halogen bedeutet.

2. Verfahren zur Herstellung der N-sulfenylierten Benzylsulfonamide der allgemeinen Formel I

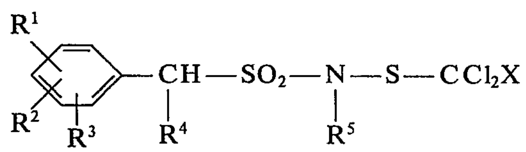

(I)

in welcher

$R^1$, $R^2$ und $R^3$ unabhängig voneinander für Wasserstoff, Halogen, Nitro, Alkyl und Halogenalkyl stehen und
$R^4$ Wasserstoff oder Alkyl bedeutet,
$R^5$ für Alkyl mit 1 bis 6 C-Atomen, Alkenyl mit 2 bis 6 C-Atomen steht, wobei diese Reste gegebenenfalls durch Heteroatome wie Sauerstoff oder Schwefel ein oder mehrmals unterbrochen sein können, Cycloalkyl mit 3 bis 7 C-Atomen, Aralkyl mit 1 bis 4 C-Atomen im Alkylteil und 6 bis 10 C-Atomen im Arylteil, Phenyl und Naphthyl steht, wobei Aralkyl, Phenyl und Naphthyl gegebenenfalls mit Halogen, Nitro, Cyano, Methyl, Trifluormethyl, Alkoxy und Alkylthio mit 1 bis 4 C-Atomen substituiert sein können und
X Halogen bedeutet,

dadurch gekennzeichnet, daß Benzylsulfonamide der allgemeinen Formel II

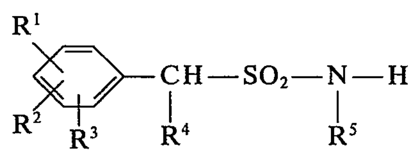

(II)

in welcher

$R^1$ bis $R^5$ die oben angegebene Bedeutung haben,

mit einem Sulfenylchlorid der Formel III

$$Cl-S-CCl_2X \quad (III)$$

in welcher

X die oben angegebene Bedeutung hat,

in Gegenwart eines säurebindenden Mittels und gegebenenfalls in Gegenwart eines Verdünnungsmittels, umgesetzt werden.

3. N-sulfenylierte Benzylsulfonamide der allgemeinen Formel I, in welcher

$R^1$, $R^2$ und $R^3$ unabhängig voneinander für Wasserstoff, Chlor, Brom, Nitro, Alkyl mit 1 bis 4 C-Atomen, Halogenalkyl mit 1 bis 4 C-Atomen und 1 bis 5 Halogenatomen stehen,
$R^4$ für Wasserstoff oder Alkyl mit 1 bis 4 C-Atomen steht und
$R^5$ für Alkyl mit 1 bis 6 C-Atomen, Alkenyl mit 2 bis 6 C-Atomen steht, wobei diese Reste gegebenenfalls durch Heteroatome wie Sauerstoff oder Schwefel ein oder mehrmals unterbrochen sein können, Cycloalkyl mit 3 bis 7 C-Atomen, Aralkyl mit 1 bis 4 C-Atomen im Alkylteil und 6 bis 10 C-Atomen im Arylteil, Phenyl und Naphthyl steht, wobei Aralkyl, Phenyl und Naphthyl gegebenenfalls mit Halogen, Nitro, Cyano, Methyl, Trifluormethyl, Alkoxy und Alkylthio mit 1 bis 4 C-Atomen substituiert sein können und
X für Fluor oder Chlor steht.

4. N-sulfenylierte Benzylsulfonamide der allgemeinen Formel I, in welcher

$R^1$ und $R^2$ unabhängig voneinander für Wasserstoff, Chlor, Nitro, Methyl und Trifluormethyl stehen,
$R^3$ für Wasserstoff steht,
$R^4$ für Wasserstoff steht und
$R^5$ für Alkyl mit 1 bis 6 C-Atomen, Alkenyl mit 2 bis 6 C-Atomen steht, die gegebenenfalls durch Heteroatome wie Sauerstoff oder Schwefel ab dem zweiten C-Atom ein- oder mehrmals unterbrochen sein können, für Cycloalkyl mit 3 bis 7 C-Atomen steht, das gegebenenfalls mit Alkyl mit 1 bis 4 C-Atomen substituiert sein kann, für Benzyl und für Phenyl steht, die beide gegebenenfalls mit Fluor, Chlor, Brom, Nitro, Cyano, Methyl, Trifluormethyl, Alkoxy und Alkylthio mit 1 bis 4 C-Atomen substituiert sein können und
X für Fluor oder Chlor steht.

5. Fungizide Mittel, gekennzeichnet durch einen Gehalt an mindestens einem N-sulfenylierten Benzylsulfonamid gemäß Anspruch 1.

6. Verfahren zur Bekämpfung von Pilzen, dadurch gekennzeichnet, daß man N-sulfenylierte Benzylsulfonamide gemäß Anspruch 1 auf Pilze oder ihren Lebensraum einwirken läßt.

7. Verwendung von N-sulfenylierten Benzylsulfonamiden gemäß Anspruch 1 zur Bekämpfung von Pilzen.

8. Verfahren zur Herstellung von fungiziden Mitteln, dadurch gekennzeichnet, daß man N-sulfenylierte Benzylsulfonamide gemäß Anspruch 1 mit Streckmitteln und/oder oberflächenaktiven Mitteln vermischt.

9. Verwendung von N-sulfenylierten Benzylsulfonamiden gemäß Anspruch 1 zum Schutz technischer Materialien.

10. Mittel zum Schutz technischer Materialien, gekennzeichnet durch einen Gehalt an mindestens einem N-sulfenylierten Benzylsulfonamid gemäß Anspruch 1.

## Claims

1. N-Sulphenylated benzylsulphonamides of the general formula I

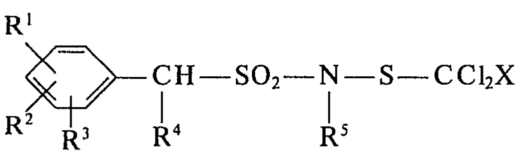

(I)

in which

$R^1$, $R^2$ and $R^3$ independently of one another represent hydrogen, halogen, nitro, alkyl or halogenoalkyl, and

$R^4$ denotes hydrogen or alkyl,

$R^5$ represents alkyl with 1 to 6 C atoms, alkenyl with 2 to 6 C atoms, it being possible for these radicals optionally to be interrupted by one or more hetero atoms such as oxygen or sulphur, cycloalkyl with 3 to 7 C atoms, aralkyl with 1 to 4 C atoms in the alkyl part and 6 to 10 C atoms in the aryl part, phenyl or naphthyl, it being possible for aralkyl, phenyl and naphthyl optionally to be substituted by halogen, nitro, cyano, methyl, trifluormethyl, alkoxy or alkylthio with 1 to 4 C atoms, and

X denotes halogen.

2. Process for the preparation of the N-sulphenylated benzylsulphonamides of the general formula I

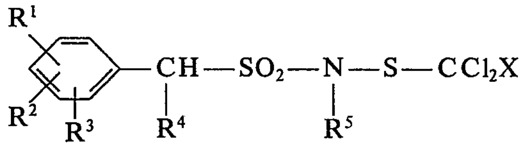

(I)

in which

$R^1$, $R^2$ and $R^3$ independently of one another represent hydrogen, halogen, nitro, alkyl or halogenoalkyl, and

$R^4$ denotes hydrogen or alkyl,

$R^5$ represents alkyl with 1 to 6 C atoms, alkenyl with 2 to 6 C atoms, it being possible for these radicals optionally to be interrupted by one or more hetero atoms such as oxygen or sulphur, cycloalkyl with 3 to 7 C atoms, aralkyl with 1 to 4 C atoms in the alkyl part and 6 to 10 C atoms in the aryl part, phenyl or naphthyl, it being possible for aralkyl, phenyl and naphthyl optionally to be substituted by halogen, nitro, cyano, methyl, trifluormethyl, alkoxy or alkylthio with 1 to 4 C atoms, and

X denotes halogen,

characterised in that benzylsulphonamides of the general formula II

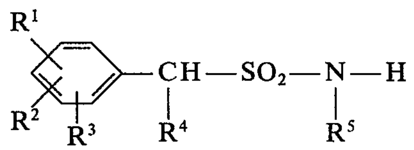

(II)

in which

$R^1$ to $R^5$ have the abovementioned meaning,

are reacted with a sulphenyl chloride of the formula III

$$Cl{-}S{-}CCl_2X \qquad (III)$$

in which

X has the abovementioned meaning,

in the presence of an acid-binding agent and if appropriate in the presence of a diluent.

3. N-Sulphenylated benzylsulphonamides of the general formula I, in which

$R^1$, $R^2$ and $R^3$ independently of one another represent hydrogen, chlorine, bromine, nitro, alkyl with 1 to 4 C atoms, or halogenoalkyl with 1 to 4 C atoms and 1 to 5 halogen atoms,

$R^4$ represents hydrogen or alkyl with 1 to 4 C atoms and

$R^5$ represents alkyl with 1 to 6 C atoms, alkenyl with 2 to 6 C atoms, it being possible for these radicals optionally to be interrupted by one or more hetero atoms such as oxygen or sulphur, cycloalkyl with 3 to 7 C atoms, aralkyl with 1 to 4 C atoms in the alkyl part and 6 to 10 C atoms in the aryl part, phenyl or naphthyl, it being possible for aralkyl, phenyl and naphthyl optionally to be substituted by halogen, nitro, cyano, methyl, trifluormethyl, alkoxy or alkylthio with 1 to 4 C atoms, and

X represents fluorine or chlorine.

4. N-Sulphenylated benzylsulphonamides of the general formula I, in which

$R^1$ and $R^2$ independently of one another represent hydrogen, chlorine, nitro, methyl or trifluoromethyl,

$R^3$ represents hydrogen,

$R^4$ represents hydrogen and

$R^5$ represents alkyl with 1 to 6 C atoms or alkenyl with 2 to 6 C atoms, which can optionally be interrupted, after the second C atom, by one or more hetero atoms such as oxygen or sulphur, cycloalkyl with 3 to 7 C atoms, which can optionally be substituted by alkyl with 1 to 4 C atoms, or benzyl or phenyl, both of which can optionally be substituted by fluorine, chlorine, bromine, nitro, cyano, methyl, trifluoromethyl, alkoxy or alkylthio with 1 to 4 C atoms, and

X represents fluorine or chlorine.

5. Fungicidal agents, characterised in that they contain at least one N-sulphenylated benzylsulphonamide according to Claim 1.
6. Method of combating fungi, characterised in that N-sulphenylated benzylsulphonamides according to Claim 1 are allowed to act on fungi or their environment.
7. Use of N-sulphenylated benzylsulphonamides according to Claim 1 for combating fungi.
8. Process for the preparation of fungicidal agents, characterised in that N-sulphenylated benzylsulphonamides according to Claim 1 are mixed with extenders and/or surface-active agents.
9. Use of N-sulphenylated benzylsulphonamides according to Claim 1 for the protection of industrial materials.
10. Agents for the protection of industrial materials, characterised in that they contain at least one N-sulphenylated benzylsulphonamide according to Claim 1.

**Revendications**

1. Benzylsulfamides N-sulfénylés de formule générale:

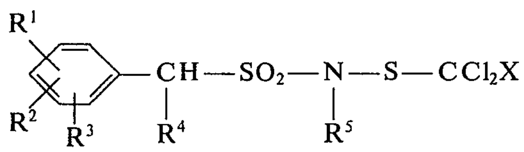

(I)

dans laquelle

$R^1$, $R^2$ et $R^3$ représentent, indépendamment l'un de l'autre, l'hydrogène, un halogène, un groupe nitro, alkyle et halogénalkyle et

$R^4$ est l'hydrogène ou un groupe alkyle,

$R^5$ est un groupe alkyle ayant 1 à 6 atomes de carbone, alcényle ayant 2 à 6 atomes de carbone, ces restes pouvant éventuellement être interrompus une ou plusieurs fois par des hétéroatomes tels que de l'oxygène ou du soufre, un groupe cycloalkyle ayant 3 à 7 atomes de carbone, aralkyle ayant 1 à 4 atomes de carbone dans la partie alkyle et 6 à 10 atomes de carbone dans la partie aryle, un groupe phényle et naphtyle, les groupes aralkyle, phényle et naphtyle pouvant éventuellement être substitués avec des radicaux halogéno, nitro, cyano, méthyle, trifluorométhyle, alkoxy et alkylthio ayant 1 à 4 atomes de carbone, et

X représente un halogène.

2. Procédé de production des benzylsulfamides N-sulfénylés de formule générale I:

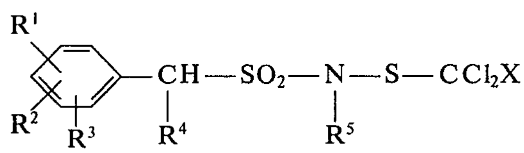

(I)

dans laquelle

$R^1$, $R^2$ et $R^3$ représentent, indépendamment l'un de l'autre, l'hydrogène, un halogène, un groupe nitro, alkyle et halogénalkyle et

$R^4$ est l'hydrogène ou un groupe alkyle,

$R^5$ est un groupe alkyle ayant 1 à 6 atomes de carbone, alcényle ayant 2 à 6 atomes de carbone, ces restes pouvant éventuellement être interrompus une ou plusieurs fois par des hétéroatomes tels que de l'oxygène ou du soufre, un groupe cycloalkyle ayant 3 à 7 atomes de carbone, aralkyle ayant 1 à 4 atomes de carbone dans la partie alkyle et 6 à 10 atomes de carbone dans la partie aryle, un groupe phényle et naphtyle, les groupes aralkyle, phényle et naphtyle pouvant éventuellement être substitués avec des radicaux halogéno, nitro, cyano, méthyle, trifluorométhyle, alkoxy et alkylthio ayant 1 à 4 atomes de carbone, et

X représente un halogène,

caractérisé en ce qu'on fait réagir des benzylsulfamides de formule générale II:

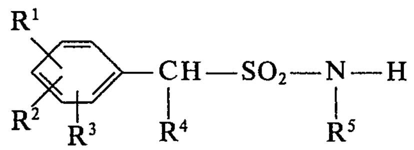

(II)

dans laquelle

$R^1$ à $R^5$ ont la définition indiquée ci-dessus,

avec un chlorure de sulfényle de formule III:

$Cl{-}S{-}CCl_2X$ (III)

dans laquelle

X a la définition indiquée ci-dessus,

en présence d'un accepteur d'acide et, le cas échéant, en présence d'un diluant.

3. Benzylsulfamides N-sulfénylés de formule générale I, dans laquelle

$R^1$, $R^2$ et $R^3$ représentent, indépendamment l'un de l'autre, l'hydrogène, le chlore, le brome, un groupe nitro, alkyle ayant 1 à 4 atomes de carbone, halogénalkyle ayant 1 à 4 atomes de carbone et 1 à 5 atomes d'halogène,

$R^4$ est l'hydrogène ou un groupe alkyle ayant 1 à 4 atomes de carbone et

$R^5$ est un groupe alkyle ayant 1 à 6 atomes de carbone, alcényle ayant 2 à 6 atomes de carbone, ces restes pouvant éventuellement être interrompus une ou plusieurs fois par des hétéroatomes tels que des atomes d'oxygène ou de soufre, un groupe cycloalkyle ayant 3 à 7 atomes de carbone, aralkyle ayant 1 à 4 atomes de carbone dans la partie alkyle et 6 à 10 atomes de carbone dans la partie aryle, un groupe phényle et un groupe naphtyle, les groupes aralkyle, phényle et naphtyle pouvant éventuellement être substitués avec un radical halogéno, nitro, cyano, méthyle, trifluorométhyle, alkoxy et alkylthio ayant 1 à 4 atomes de carbone et

X représente le fluor ou le chlore.

4. Benzylsulfamides N-sulfénylés de formule générale I, dans laquelle

$R^1$ et $R^2$ représentent, indépendamment l'un de l'autre, l'hydrogène, le chlore, les groupes nitro, méthyle et trifluorométhyle,

$R^3$ est l'hydrogène,

$R^4$ est l'hydrogène et

$R^5$ est un groupe alkyle ayant 1 à 6 atomes de carbone, alcényle ayant 2 à 6 atomes de carbone, qui peuvent éventuellement être interrompus une ou plusieurs fois par des hétéroatomes tels que des atomes d'oxygène ou de soufre à partir du second atome de carbone, un groupe cycloalkyle ayant 3 à 7 atomes de carbone, qui peut être substitué, le cas échéant, avec un groupe alkyle ayant 1 à 4 atomes de carbone, un groupe benzyle et un groupe phényle, tous deux pouvant éventuellement être substitués avec du fluor, du chlore, du brome, un radical nitro, cyano, méthyle, trifluorométhyle, alkoxy et alkylthio ayant 1 à 4 atomes de carbone et

X représente de fluor ou le chlore.

5. Composition fongicide, caractérisée par une teneur en au moins un benzylsulfamide N-sulfénylé suivant la revendication 1.

6. Procédé pour combattre des champignons, caractérisé en ce qu'on fait agir des benzylsulfamides N-sulfénylés suivant la revendication 1 sur des champignons ou sur leur milieu.

7. Utilisation de benzylsulfamides N-sulfénylés suivant la revendication 1 pour combattre des champignons.

8. Procédé de préparation de compositions fongicides, caractérisé en ce qu'on mélange des benzylsulfamides N-sulfénylés suivant la revendication 1, avec des diluants et/ou des agents tensio-actifs.

9. Utilisation de benzylsulfamides N-sulfénylés suivant la revendication 1, pour la protection de matériaux industriels.

10. Composition pour la protection de matériaux industriels, caractérisée par une teneur en au moins un benzylsulfamide N-sulfénylé suivant la revendication 1.